# EUROPÄISCHE PATENTANMELDUNG

(11) **EP 1 627 649 A1**
(43) Veröffentlichungstag der Anmeldung: **22.02.2006**
(21) Anmeldenummer: 05105558.0
(22) Anmeldetag: 22.06.2005
(51) Int. Cl.: A61L 15/18, A61L 26/00

(54) **Magnesiumhaltiges Wundauflagematerial**

(30) Priorität: 20.07.2004 DE 102004035905
(71) Anmelder: Biotronik V1 Patent AG, 6340 Baar (CH)
(72) Erfinder: Kuttler, Marc, Dr., 12205, Berlin (DE)
(74) Vertreter: Eisenführ, Speiser & Partner

(57) **Zusammenfassung**

Die Erfindung betrifft ein biokompatibles und bioabsorbierbares Wundauflagematerial. Der vorliegenden Erfindung liegt die Aufgabe zugrunde, die Wundversorgung durch ein biokompatibles, biodegradierbares Wundauflagematerial zu verbessern. Körpereigenen Abwehr- und Heilungsmechanismen sollen dabei unterstützt und antimikrobielle und antiproliferative Wirkungen erzielt werden. Dies wird dadurch erreicht, dass das Wundauflagematerial elementares Magnesium enthält.

## Beschreibung

Die Erfindung betrifft Wundauflagematerialien, die zur Behandlung von infizierten Wunden, anderen entzündlichen Hauterkrankungen oder zum vorbeugenden Schutz vor Wundinfektionen eingesetzt werden können und den Heilungsprozess fördern.

Die Behandlung und Heilung von infizierten bzw. infektionsgefährdeten Wunden von Mensch oder Tier - hervorgerufen durch Verletzungen, Verbrennungen, operative Eingriffe (akute Wunden), allergische Reaktionen oder andere Hauterkrankungen und insbesondere auch von chronischen Wunden - ist ein primäres Ziel moderner Medizintechnik. Eine übermäßige Besiedlung des Wundmilieus mit Mikroorganismen wie z.B. Bakterien der Gattungen Pseudomonas oder Staphylococcus kann zu einer massiven Störung des Heilungsprozesses bis hin zur Letalität führen. Eine Eingrenzung der Gefahr auf wenige Arten ist nicht möglich, da viele der Mikroorganismen als opportunistische Krankheitserreger anzusehen sind.

Eine Vielzahl von Methoden ist bekannt, um Krankheitserreger aus infiziertem Gewebe zu entfernen oder abzutöten.

Stark verbreitet ist z.B. der Einsatz von Antibiotika, die auf die Wunde aufgebracht werden oder aber oral bzw. intravenös verabreicht werden. Der weit verbreitete Einsatz von Antibiotika hat jedoch zur Ausbildung von Resistenzen bei vielen Krankheitserregen geführt. So sind inzwischen ca. 23% aller Staphylococcus Stämme resistent gegenüberherkömmlichen einem oder mehreren Antibiotika wie z.B. Penizillin.

Eine andere Möglichkeit der Wundversorgung ist die mechanische Reinigung mit Ringer-Lösung, einem sehr zeitaufwändigen Verfahren, das auch den Heilungsprozess verzögert.

Zur antimikrobiellen Therapie von Wunden oder zur Vorbeugung allgemein bekannt ist auch die Verwendung von Oxidantien (z.B. Jodtinktur) oder Antiseptika (z.B. Silbersulfadiazin). Metall-Ionen, insbesondere Edelmetall-Ionen wie Gold- und Silber-Ionen, werden seit vielen Jahren in der Wundversorgung zur Vermeidung und Bekämpfung von Infektionen eingesetzt. Besonders hervorzuheben ist das allbekannte Silbernitrat. So wird z.B. 5%ige Silbernitrat-Lösung bisweilen für die Behandlung von Verbrennungen 2ten Grades verwendet, die bis zu 12 mal pro Tag auf den Wundbereich aufgebracht werden muss, um ausreichend aktive Ionen bereitzustellen. Bei der Verwendung von silberhaltigen Cremes (z.B. Silbersulfadiazin) wiederum sind die Rückstände regelmäßig zu entfernen, um allergische Reaktionen zu vermeiden.

Zum Einsatz von Silberverbindungen bei der Wundbehandlung sind viele Ausführungsformen wie Pulver, Flocken, Beschichtungen, Folien, Fasern, Filme, Papier oder Kunststoffe (JP 03083905, JP 60 181 002, US 5,454,886) bekannt.

In der modernen Wundbehandlung werden heutzutage auch hydrophobierte Trägermaterialien (EP 0021230 B1; EP 0162026 B1; US A 4,393,048; EP 0475807 B1) verwendet, um ein Austrocknen der Wunde zu vermeiden und so die natürlichen Abläufe bei der Wundheilung zu unterstützen. Auch hier finden Silberverbindungen wegen der antimikrobiellen Wirkung Anwendung.

Festzuhalten bleibt aber bei all diesen Ausführungsformen, dass es zu einem direkten Kontakt des Wundgewebes mit Silberionen kommt und so auch gesundes Zellwachstum während der Wundheilung negativ beeinflusst wird.

Die bei der Wundversorgung verwendeten Wundauflagematerialien, die unter anderem die Wunde vor äußeren Einflüssen und weiteren Verunreinigungen/Kontaminierung schützen, sind üblicherweise nicht absorbierbare Materialien, die nicht in der Wunde verbleiben dürfen. Solche im Kontakt mit der Wunde stehenden Wundauflagen bzw. Verbände verkleben häufig mit der Wunde. Ein entfernen/erneuern der Wundauflage ist für den Patienten traumatisch, verzögert durch ein erneutes Aufreißen der Wunde den Heilungsprozess und steigert die Gefahr einer erneuten Infektion.

Inzwischen hat man begonnen bioresorbierbare Materialien zu entwickeln, die in der Wunde verbleiben können und ein einwachsen/verkleben mit den übrigen Wundauflageschichten vermeiden. Sie dienen auch als Träger für antimikrobielle Substanzen, sollen die Wunde auffüllen und bis zur ausreichende Verheilung schützen. Ein solches bioresorbierbares Material ist z.B. Collagen, dass u.a. in Form von Perlen eingesetzt wird (DE 697 14 226 T2). Solche bioresorbierbaren Materialen sind jedoch aufwendig in Herstellung und Sterilisation. Für eine mikrobielle Wirkung oder um auch unkontrolliertes Zellwachstum zu verhindern, welches im unkritischsten Fall zumindest eine unerwünscht starke Narbenbildung hervorruft, müssen weitere pharmazeutische Wirkstoffe eingesetzt werden.

Auch wird die Aktivierung und Unterstützung der körpereigenen Heilungsmechanismen für einen schnellen Heilungsverlauf bei den herkömmlichen Wundversorgungsverfahren nur sehr bedingt verfolgt.

Das nachveröffentlichte deutsche Patent DE 103 16 153 B3 beschreibt ein Polymermaterial als Verbands- oder Wundauflagenmaterial, das antimikrobiell wirksames silberhaltiges Glas enthält. Dem Material kann elementares Magnesium zugesetzt werden.

Der vorliegenden Erfindung liegt die Aufgabe zugrunde, die Wundversorgung durch ein biokompatibles, biodegradierbares Wundauflagematerial zu verbessern. Körpereigene Abwehr- und Heilungsmechanismen sollen dabei unterstützt und antimikrobielle und antiproliferative Wirkungen erzielt werden.

Erfindungsgemäß wird dies dadurch erreicht, dass das Wundauflagematerial elementares Magnesium enthält. Das elementare Magnesium liegt dabei in Form einer biodegradierbaren Magnesiumlegierung vor. Die zu behandelnde Wunde wird zumindest teilweise mit Magnesium in Form einer Legierung bedeckt oder das Magnesium wird in Form einer Legierung zumindest teilweise in die Wunde eingebracht.

Unter "Biodegradation" werden hydrolytische, enzymatische und andere stoffwechselbedingte Abbauprozesse im lebendem Organismus verstanden, die zu einer allmählichen Auflösung zumindest großer Teile der verwendeten Materialien führen. Synonym wird häufig der Begriff Biokorrosion verwendet. Der Begriff Bioresorption umfasst zusätzlich die anschließende Resorption der Abbauprodukte.

Es ist allgemein hin bekannt, dass Magnesiumionen ein unentbehrlicher und vielseitiger Aktivator lebenswichtiger Prozesse sind.

Im Hinblick auf antimikrobielle Wirkungen vom Magnesiumionen weiß man, dass die unspezifische Abwehr über das Properdinsystems nur bei Anwesenheit von Magnesiumionen wirksam ist und die Phagozytose der Bakterien durch Leukozyten eine Anregung durch das Magnesiumion erfährt. Somit sorgen Magnesiumionen u.a. für die Bekämpfung von Infektionen durch Unterstützung bzw. Aktivierung des körpereigenen Immunsystems und mindert auch generell die Anfälligkeit für Infektionen (Dr. med. Dr. sc. Nat PG Seeger, SANUM-Post Nr. 13/1990, 14-16).

Bezüglich der Wundheilung sind Magnesiumionen für den anaeroben Stoffwechsel notwendig und fördert die normale Granulation des Bindegewebes, d.h. eine schnelle Wundheilung durch das Auffüllen der Wunde mit gesundem Gewebe. Bei Verbrennungen wirkt sich die Gegenwart von Magnesiumionen positiv aus, da es den Ammoniakspiegel senkt und somit Hautfunktionen im Verbrennungsbereich unterstützt.

Die Anwendung von Magnesium-Legierungen (die elementares Magnesium enthalten) im Rahmen der Wundversorgung, insbesondere deren direkte Anwendung, ist bislang unbekannt. Eine Darreichung erfolgt bisher typisch in oraler oder intravenöser Form, meist als Ergänzung zur Ernährung um vielfältige Körperfunktionen zu unterstützen, wobei das Magnesium jedoch immer in ionischer Form vorliegt.

Es hat sich nun in in-vivo- und in-vitro-Versuchen überraschender Weise gezeigt, dass die Verwendung von elementarem Magnesium in Magnesium-Legierungen geeigneter Zusammensetzung einer starken immunologischen Reaktionen des Körpers entgegenwirken. Auch eine antiproliferative Wirkung insbesondere auf glatte Muskelzellen und Endothel-Zellen konnte in in-vitro-Versuchen belegt werden, d.h. Wucherungen die die Quelle starker Vernarbungen sind, könnten verhindert bzw. stark eingedämmt werden, ein kontrolliertes Zellwachstum findet jedoch statt.

Das elementare Magnesium liegt in Form einer biodegradierbaren Magnesiumlegierung vor, über deren Komposition sich das Biodegradationsverhalten, d.h. insbesondere der Degradationszeitraum, die mechanischen Eigenschaften, sowie die biologische Wirksamkeit steuern lassen, wie die Materialuntersuchungen sowie die in-vivo und in-vitro-Tests gezeigt haben. Die Magnesiumlegierung sollte mindestens 50 Gew.%, vorzugsweise mindestens 70 Gew.%, besonders bevorzugt mindestens 85 Gew.% Magnesium enthalten.

Der den positiven Effekten zugrunde liegende Wirkungsmechanismus ist bisher in Detail nicht ergründet. Die Korrelation mit den im Allgemeinen bekannten Wirkungen des Magnesiums lassen jedoch vermuten, dass auch bei der direkten Verwendung von elementarem Magnesium und Magnesium-Legierungen die bei der Degradation vom Körper aufgenommenen Zerfallsprodukte zumindest lokal zum Teil die gleichen Prozesse im Körper aktivieren, wie das z.B. mit der Nahrung aufgenommene ionische Magnesium. Auch eine direkte antibakterielle Wirkung ist nicht auszuschließen. Auch scheinen die Abbauprozesse der Legierung einen Einfluss auf das Geschehen zu haben.

Nach einer bevorzugten Variante der vorgenannten Ausführungsform wird die spezifische Zusammensetzung der Magnesiumlegierung sowie seine Modifikation dahingehend vorgegeben, dass die Zersetzung unmittelbar nach der Aufbringung einsetzt und so lange Aufrecht erhalten wird, bis zumindest teilweise die Wunde mit Bindegewebe erfüllt ist. Dieser Zeitraum sollte vorzugsweise zwischen 1h bis 60 Tage, insbesondere 1 bis 10 Tage, betragen. Der Umfang der Abbauprozesse ist von den am Anwendungsort herrschenden Bedingungen abhängig.

Es ist denkbar, durch die gezielte Beimischung geeigneter Metalle wie z.B. Silber, Gold oder Seltener Erden die antibakterielle Wirkung zu verstärken, ohne die Biokompatibilitäts- und Degradationseigenschaften zu verschlechtern.

Besonders bevorzugt sind biodegradierbare Magnesium-Legierungen, die Seltenerdmetalle und Yttrium enthalten (WE-Magnesiumlegierungen), wobei die Sammelbezeichnung ,Seltenerdmetall' für die Elemente Scandium (Ordnungszahl 21), Lanthan (57) sowie die 14 auf das Lanthan folgenden Elemente Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), die als Lanthanoide bezeichnet werden, steht. Besonders bevorzugt weisen die Magnesiumlegierungen folgende Gewichtsanteile der Legierungskomponenten auf:
- Seltenerdmetalle 2.0 bis 5.0 Gew.% und/oder
- Yttrium 3.5 bis 4.5 Gew.% und/oder
- Neodym 1.5 bis 3.0 Gew.% und/oder
- Zirkonium 0.3 bis 1.0 Gew.% und/oder
- Aluminium < 0.5 Gew.%, insbesondere < 0.01 Gew.%, und/oder
- Rest < 0.5 Gew.%, insbesondere < 0.3 Gew.%,

wobei Magnesium den auf 100 Gew.% verbleibenden Gewichtsanteil an der Legierung einnimmt. Die genannten Magnesiumlegierungen zeigen ein günstiges Abbauverhalten, hohe Biokompatibilität der Legierung als auch der Abbauprodukte und besitzen für das Einsatzgebiet ausreichende mechanische Eigenschaften.

Für eine effiziente Anwendung sind unterschiedliche Ausführungsformen denkbar. Je nach Wundengröße, Tiefe und Art sind Pulver bzw. Körner, Fäden bzw. Draht, Späne oder Folien zu bevorzugen. So sind Pulver bzw. Körner am einfachsten bei beliebigen Wundengrößen anwendbar, Fäden und Draht sind eventuell besser für tiefe Wunden geeignet, während eine Folie gut für großflächige Verletzungen geeignet ist, bei denen auch eine gute Schutzfunktion gegen äußere Einflüsse von Vorteil ist, wie z.B. bei Brandverletzungen. Statt einer Folie können auch Gewebe die aus Fäden gepresst oder gewoben werden, verwendet werden.

Das magnesiumhaltige Material kann direkt auf die Wunde aufgetragen werden, in eine weitere Wundauflage eingebettet bzw. aufgebracht sein, oder auch in eine Salbe oder Spülung eingebracht sein.

Das magnesiumhaltige Material kann auch als Trägermaterial für weitere pharmazeutische Wirkstoffe verwendet werden.

Der direkte Kontakt des magnesiumhaltigen Materials mit der Wunde sollte jedoch sichergestellt sein, um eine optimale Wirkung des Magnesiums und ein Separation von Wunde und nicht resorbierbaren Materialien zu erhalten.

Da Magnesium und die Magnesium-Legierungen einen sehr hohen Umformungsgrad haben, ist eine Verarbeitung über herkömmliche Materialverarbeitungsverfahren kein Problem. Barren-, Rohr-, Span-, Draht- oder Pulverausführungen sind am Markt beziehbar (BDI-Einkaufsführer, Deutsche Industrie). Herkömmliche Folien sind über Walzverfahren herstellbar. Auch extrem dünne Magnesium-Folien (µm), in der Vergangenheit über Hämmer-Verfahren hergestellt, lassen sich heute über Pulverbeschichtungsverfahren herstellen.

Beschichtungen von Trägermaterialien lassen sich z.B. über Pulverbeschichtung oder Sputtern realisieren.

Das Material lässt sich insbesondere mittels Gamma- oder Beta-Bestrahlung oder auch mit alkoholischen Lösungen sterilisieren.

## Patentansprüche

1. Wundauflagematerial, enthaltend elementares Magnesium, **dadurch gekennzeichnet, dass** das Magnesium in Form einer biodegradierbaren Legierung vorliegt.

2. Wundauflagematerial nach Anspruch 1, **dadurch gekennzeichnet, dass** die Magnesiumlegierung eine Legierung des Typs WE ist.

3. Wundauflagematerial nach einem der vorhergehenden Ansprüche, **dadurch gekennzeichnet, dass** eine spezifische Zusammensetzung der Magnesiumlegierung sowie seine Modifikation dahingehend vorgegeben sind, dass eine Zersetzung unmittelbar nach der Auftragung einsetzt und so lange Aufrecht erhalten wird, bis zumindest bereichsweise die Wunde mit Bindegewebe bedeckt ist.

4. Wundauflagematerial nach Anspruch 1, **dadurch gekennzeichnet, dass** das magnesiumhaltige Material in Form eines Pulvers vorliegt.

5. Wundauflagematerial nach Anspruch 1, **dadurch gekennzeichnet, dass** das magnesiumhaltige Material in einen Wundverband integriert ist.
